# EUROPEAN PATENT SPECIFICATION

(11) **EP 1 567 047 B1**
(45) Date of publication and mention of the grant of the patent: **19.11.2008**
(21) Application number: 03748867.3
(22) Date of filing: 10.10.2003
(51) Int. Cl.: A61B 1/253

(54) **DENTIST'S MIRROR**
ZAHNARZTSPIEGEL
MIROIR DE DENTISTE

(30) Priority: 14.10.2002 SE 0203049; 11.11.2002 SE 0203312
(43) Date of publication of application: 31.08.2005
(73) Proprietor: Kent Nyman I Göteborg AB, 41136 Göteborg (SE)
(72) Inventor: NYMAN, Kent, Kent Nyman i Göteborg AB, 411 36 Göteborg (SE)
(74) Representative: Vink, Charlotta
(86) International application number: PCT/SE2003/001578
(87) International publication number: WO 2004/034892

(56) References cited:
- EP-A1- 0 314 657
- FR-A1- 2 642 298
- US-A- 2 228 169
- US-A- 2 393 319
- US-A- 3 859 987
- US-A- 5 951 284

## Description

### Technical Field of Invention

The present invention relates to a dental instrument comprising a mirror and a suction device having an inlet through which liquid is sucked by the suction device from the oral cavity of the patient.

### Background of the Invention

For dental operations, such as fillings of cavities, and the like on the patient's teeth the dentist needs, in addition to drilling equipment and a mirror, also a suction device to suck up the flushing fluid emanating from the drilling equipment, the saliva of the patient, and tooth and filling debris, if any. The mirror is used to keep the tongue and the cheeks of the patient away from the tooth being operated on while at the same time it reflects light onto the working location from an external source of light, thus allowing the dentist a good view of the working area. These three devices, drill, mirror and suction device, cannot be held and manipulated simultaneously by one single person and for this reason the dentist normally needs the assistance of a dental nurse. The dentist manipulates the drilling equipment as well as the mirror while simultaneously the dental nurse manipulates the suction device. The task of the dental nurse is to direct the suction device in such a manner that liquid being sucked from the drilling equipment flows on top of part of the mirror, ensuring that the latter is kept clean during the operation. If for some reason or other no dental nurse is present, the mirror is omitted and the patient is put at an angle that enables the dentist, although with some difficulty, to view the intended working area and operate without using a mirror. However, in cases like these the dentists has to work while assuming a very uncomfortable position, and at the same time he has to interrupt the operation at regular intervals in the first place to check the working area by means of a mirror and in the second place to suck up liquid from further down the patient's pharynx. As a consequence, the operation is more time-consuming than is really necessary.

Suction devices conventionally consist of a straight tubular suction nozzle having a diameter of about 1 cm, which is connected to a pump and to an outlet for removal of the sucked-up liquid. A conventional dentist's mirror is circular and has a diameter of about 2.2 cm. The field of vision that is provided by the mirror of the mirror is, however, restricted to about 20% of the area of the mirror because of the latter being covered by saliva, flushing liquid and rests of the preparations being used in the course of the operation.

Some solutions to the problem connected with covered mirrors suggested by prior-art devices on the market involve attachment of a conventional mirror to a suction nozzle. A solution of this nature is presented e.g. in FR 2 595 939. However, in this case the suction nozzle is spaced too far away from the point of surgery in the oral cavity since it is obstructed by the mirror, and as a result the suction force is heavily reduced. In other prior-art devices, the suction nozzle extends in a channel formed in the mirror handle and debouches either in the rear edge or the front edge of the mirror, or at the back of the mirror. This arrangement reduces the suction capacity considerably while at the same time the channels in which the liquid is to be conveyed are narrow with consequential risks that particles from the oral cavity, such as bits of amalgam fillings or bits of other removed material, stuck in the channels.

Document EP-A-0314657 discloses a dental instrument according to the preamble of claim 1.

By liquid is to be understood herein the mixture of liquid, air and debris of teeth and fillings found in the oral cavity during a dental treatment.

### Summary of the Invention

The object of the invention thus is to remedy the problem outlined above. This object is achieved in accordance with invention by means of a dental instrument of the kind defined in the introduction, which has been given the characteristic features defined in claim 1. Preferred embodiments of the invention are described in the claims dependent on claim 1.

The present invention relates to a dental instrument comprising a mirror and a suction device having an inlet. Liquid originating from the patient's oral cavity is sucked up by the suction device via the inlet. The reflecting surface of the mirror is passed through by an opening, which during use of the instrument forms the mouth of said inlet and the reflecting surface of the mirror is flushed clean by the liquid flowing across it. In an instrument thus constructed the suction device and the mirror may be formed as one single unit or as separate units that are assembled prior to use. The mirror may for instance be formed with a centrally located through-opening and during use the mirror is placed on the inlet of the suction device in a manner allowing flushing liquid emanating e.g. from the drilling device to be sucked across the mirror surface when the suction device is in operation and liquid is present in the oral cavity during use of the drilling equipment, and to pass across the mirror surface. In this manner, the mirror becomes self-cleaning and condensation-free. In addition, one hand of either the dentist or the nurse is freed because of the reduced number of devices needed for the treatment of the patient. An additional advantage found in the arrangement of the mirror around the inlet of the suction nozzle is that the flow of medium of the suction device will come closer to the area being treated than is the case in mirrors the surface of extension of which is in alignment with the direction of the suction device. Furthermore, it becomes possible to reduce the area of the mirror because a larger portion of its reflecting surface is kept clean than is the case in prior-art mirrors. An added advantage is that the suction capacity of the suction device is maintained, since the size of the inlet need not be reduced. Unlike in prior-art devices featuring a combination of the function of the suction device and the function of the mirror, the present invention focuses on the function of the suction device while the prior-art focuses on the function of the mirror. As a result, the present invention is superior in meeting the functional demands normally put on suction devices.

Preferably, during use the mirror is prevented from rotating in the inlet of the instrument. If the mirror were to rotate, the field of vision that it provides would be lost and the operation would have to be interrupted with consequential time loss and possible injuries to the patient.

It is likewise suitable to arrange for said mirror to suck up, through said opening, all liquid intended to be removed. Preferably, the mirror forms a tight seal against the suction devise to ensure that no liquid is sucked into the instrument behind the mirror. A different arrangement would considerably reduce the mirror's cleaning ability.

Furthermore, the mirror advantageously extends in the manner of a flange away from said inlet. Owing to the flange-like extension of the mirror away from the inlet and the angle of the mirror relative to the suction nozzle and the inlet therein, the reflecting area of the mirror could be given a shape and a configuration that is suitable considering the intended use and in addition the suction function could be maintained at a powerful level and be effective close to the area under treatment without interference from the mirror.

It is likewise advantageous that the mirror forms an integrated part of the instrument. In this preferred embodiment the suction nozzle and the mirror therefore forms one single unit. The instrument thus becomes easy to manipulate and its function is ensured, since already from the start it possesses the qualities required to provide optimum function.

In accordance with another suitable embodiment the mirror is removably attachable to the instrument. In this case the instrument consists of a separate suction nozzle and a separate mirror and the latter is attached to the suction-nozzle inlet prior to use. The mirror may be attached to a prior-art suction nozzle in any known manner. Suction devices already existing on the market thus may be used, making them more versatile. At the same time they benefit from the advantages provided by the invention but to a low price. A set of differently designed mirrors may be provided, thus offering the dentist the opportunity to change mirrors also during the ongoing treatment of a patient.

Advantageously, the mirror of the instrument is of a magnifying kind, i.e. it preferably is concave in order to further enhance the dentist's vision.

In accordance with yet another advantageous embodiment the suction device comprises a tubular suction nozzle at the inlet of which said opening in the mirror forms a mouth and the suction nozzle is curved. The reason for the curvature of the tubular suction nozzle is to set the mirror at an angle and consequently to increase the dentist's vision. Parts of the suction nozzle may change it's angular direction once or several times or may have a gradually transitional change of it's angular direction.

It is likewise advantageous to arrange said mirror removably attachable to said suction device by means of a frictional coupling arrangement. This could be achieved for instance by providing a frictional coupling on the inner or the external face of the nozzle. This makes it an easy task to exchange the mirror and at the same time the coupling forms a tight transitional seal between the suction nozzle and the mirror.

In accordance with yet another preferred embodiment the mirror is by means of a holder removably attachable to a seat formed in said suction device. Consequently, the suction nozzle may be provided with an inlet, the edge of which forms a seat to which the mirror is intended to be fastened. In the case when the mirror is a separate part of the instrument, the same suction device may be used together with mirrors of various types or shapes, thus enhancing the usefulness of the instrument further.

### Brief Description of the Drawings

The invention will be described in the following in more detail with reference to the accompanying drawings that for purely exemplifying reasons show presently particularly preferred embodiments of the present invention. In the drawings:
Fig 1 is a view from above of en instrument in accordance with the invention,
Fig 2 is a longitudinal sectional view through the instrument of Fig 1,
Fig 3 is a longitudinal sectional view through an alternative embodiment of an instrument in accordance with the invention,
Fig 4 is a longitudinal sectional view through an alternative embodiment of an instrument in accordance with the invention,
Fig 5 is a longitudinal sectional view through an alternative embodiment of an instrument in accordance with the invention,
Fig 6 is a view from above of an alternative embodiment of an instrument in accordance with the invention, and
Fig 7 is a longitudinal sectional view through an alternative embodiment of an instrument in accordance with invention.

References to the components shown in the drawing figures and having identical functions have received the same numeral references.

### Detailed Description of Preferred Embodiments

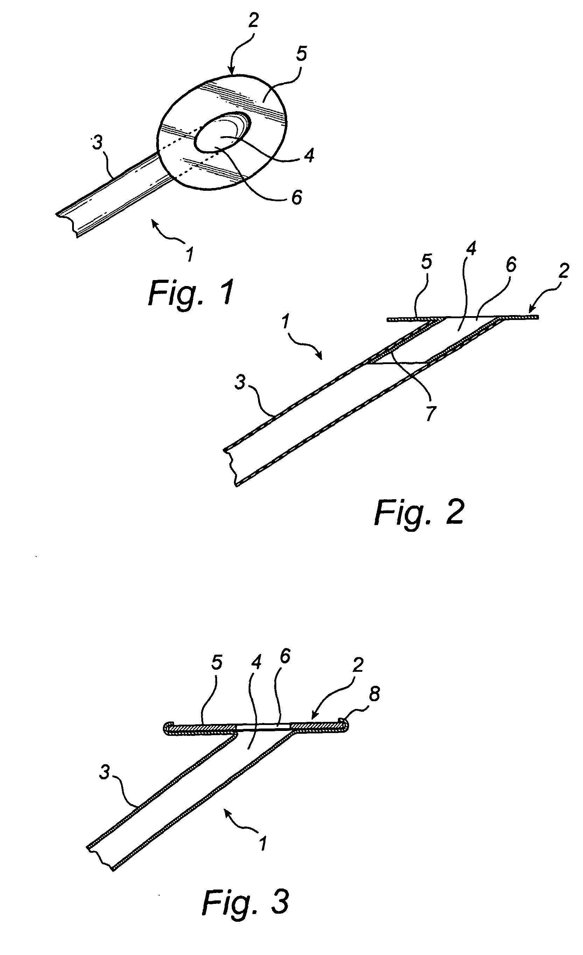

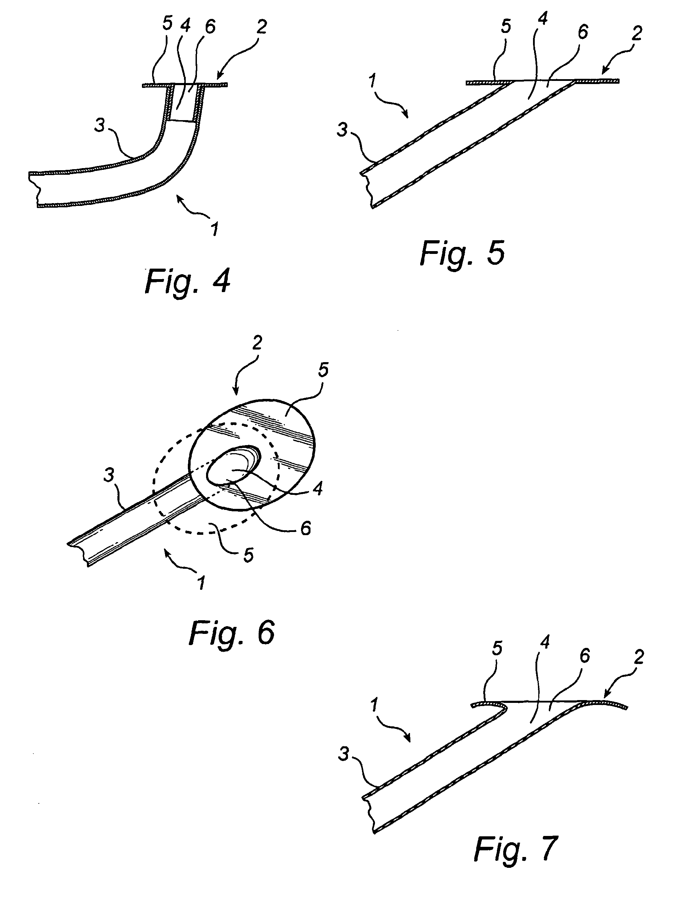

A dental instrument 1 in accordance with the present invention comprises a mirror 2 and a suction device 3. The suction device 3 has an inlet 4 through which liquid emanating from the patient's oral cavity is sucked up during active use of the instrument 1. The part of the suction device 3 that is used in the patient's cavity consists of a tubular suction nozzle, which could either be straight (see for example Fig 2) or somewhat curved (see Fig 4) for adaptation to the configuration of the area of the cavity where it is to be used. The suction nozzle of the suction device 3 has a shape in the area of its inlet 4 such that said inlet extends at an angle to the lengthwise extension of the suction nozzle. Like the curve of the suction nozzle also the angle could be adapted to the configuration of the area of the cavity where the instrument 1 is to be used. The reflecting face 5 of the mirror 2 is turned away from the suction nozzle and from the inlet 4 of said nozzle. The mirror 2 extends like a flange outwardly away from the inlet 4 and encloses the entire inlet 4. An opening forming the mouth 6 of the suction device 3 passes through the reflecting face 5 of the mirror 5. The mirror 2 is attached tightly to the inlet 4 preventing any liquid from being sucked into the instrument 1 behind the mirror. Figs 1 and 2 show an embodiment according to which the mirror 2 is connected to the inlet by means of a frictional coupling 7. The frictional coupling 7 extends like a sleeve from the mirror 2 so as to project into the interior of the inlet 4. In accordance with another embodiment illustrated in Fig 3, the mirror 2 instead consists of a flat reflecting face 5, which is attached to an inlet 4, the latter being enlarged so as to present a seat to which the mirror 2 may be attached. The seat may be pressed outwards and be widened somewhat in its circumferential direction as pressure is being exerted thereon, in order to accommodate the mirror 2. Once the mirror 2 is in place, the seat resumes its original shape and keeps the mirror 2 in place. In a corresponding manner, the mirror 2 may be released by the seat being again pressed outwards, allowing the mirror to be removed 2. In this manner, mirrors 2 of different sizes and/or shapes may be used, according to need.

When the instrument 1 is to be used, the mirror 2 is attached to the inlet 4. The instrument is then introduced into the patient's oral cavity and is directed towards the area to be treated. While the suction device 3 sucks up liquid emanating for instance from a drilling device the mirror 2 is simultaneously cleaned by the current of air that is generated by the suction device, and thus liquid is caused to flow across the surface of the mirror 2, which thus is kept clean and moisture-free.

As should be appreciated, numerous modifications of the embodiments described above are possible within the scope of protection of the invention. As already mentioned the suction nozzle in accordance with Fig 4 may have a curved configuration to facilitate operations performed in different areas of the patient's oral cavity. In addition, the instrument 1 could be made as a single unit by designing the mirror as an integral part of the suction nozzle of the suction device 3. An embodiment of this kind is shown in Fig 5 and Fig 7. Fig 7 also shows an example of a mirror 2 that may have a slightly concave shape to provide a magnifying effect. The mirror 2 may also have a rectangular shape or be formed with several facets, which may be either straight or have some other configuration. The various facets may be arranged in such a mutual relationship that together they form a funnel-like mouth in the suction device, thus further enhancing the instrument's liquid-collecting capacity. The friction coupling 7 could consists of prongs projecting into the interior of the inlet 4 to keep the mirror in place while at the same time preventing the latter from rotating. Furthermore, Fig 6 shows an example of adaptation of the external shape of the mirror 2 to the current need. Such adaptation of shape could of course also be made in the case of integrated as well as replaceable mirrors 2. The configuration indicated in Fig 6 in broken lines is but one further example of one manner of such shape adaptation.

In addition, the components making up the device could be produced as disposable articles or as reusable articles that lend themselves to sterilisation.

## Claims

1. A dental instrument (1) comprising a mirror (2) and a suction device (3) having an inlet (4) through which liquid is sucked by said suction device, the reflecting surface of the mirror (2) thus being capable of being flushed clean by the liquid flowing across it, **characterised in that** said inlet (4) passes through the reflecting surface (5) of the mirror (2).

2. A dental instrument as claimed in claim 1, wherein said mirror (2) is prevented from rotating during use.

3. A dental instrument as claimed in any one of the preceding claims, wherein said mirror (2) extends as a flange away from said inlet.

4. A dental instrument as claimed in any one of the preceding claims, wherein said mirror (2) forms an integrated part of said instrument (1).

5. A dental instrument as claimed in an one of the preceding claims, wherein said mirror (2) is removably attachable to said instrument (1).

6. A dental instrument as claimed in any one of the preceding claims, wherein said mirror (2) is a magnifying mirror.

7. A dental instrument as claimed in any one of the preceding claims, wherein said suction device (3) comprises a tubular suction nozzle, the inlet of which forms a mouth (6), and wherein said suction nozzle is curved.

8. A dental instrument as claimed in claim 3, wherein said mirror (2) is removably attached to said suction device (3) by means of a frictional coupling arrangement (7).

9. A dental instrument as claimed in claim 3, wherein said mirror (2) is removably attached to a seat in said suction device (3) by means of a holder.

## Patentansprüche

1. Zahnärztliches Instrument (1), das einen Spiegel (2) und eine Saugvorrichtung (3) umfasst, die einen Einlass (4) aufweist, durch den mittels der Saugvorrichtung Flüssigkeit gesaugt wird, wobei die reflektierende Fläche (5) des Spiegels (2) auf diese Weise durch die über sie hinweg fließende Flüssigkeit sauber gespült werden kann, **dadurch gekennzeichnet, dass** der Einlass (4) durch die reflektierende Fläche (5) des Spiegels (2) hindurch verläuft.

2. Zahnärztliches Instrument nach Anspruch 1, wobei verhindert wird, dass sich der Spiegel (2) während des Gebrauchs drehen kann.

3. Zahnärztliches Instrument nach einem der vorangehenden Ansprüche, wobei sich der Spiegel (2) als ein Flansch von dem Einlass fort erstreckt.

4. Zahnärztliches Instrument nach einem der vorangehenden Ansprüche, wobei der Spiegel (2) einen integrierten Teil des Instruments (1) bildet.

5. Zahnärztliches Instrument nach einem der vorangehenden Ansprüche, wobei der Spiegel (2) abnehmbar an dem Instrument (1) angebracht werden kann.

6. Zahnärztliches Instrument nach einem der vorangehenden Ansprüche, wobei der Spiegel (2) ein Vergrößerungsspiegel ist.

7. Zahnärztliches Instrument nach einem der vorangehenden Ansprüche, wobei die Saugvorrichtung (3) eine röhrenförmige Saugdüse umfasst, deren Einlass eine Mündung (6) bildet, und wobei die Saugdüse gekrümmt ist.

8. Zahnärztliches Instrument nach Anspruch 3, wobei der Spiegel (2) mit Hilfe einer Reibungskupplungsanordnung (7) abnehmbar an der Saugvorrichtung (3) angebracht ist.

9. Zahnärztliches Instrument nach Anspruch 3, wobei der Spiegel (2) mit Hilfe einer Halterung abnehmbar an einem Sitz in der Saugvorrichtung (3) angebracht ist.

## Revendications

1. Instrument dentaire (1) comprenant un miroir (2) et un dispositif d'aspiration (3) comportant un orifice d'entrée (4) à travers lequel un liquide est aspiré par ledit dispositif d'aspiration, la surface réfléchissante du miroir (2) étant ainsi en mesure d'être rincée par le liquide s'écoulant à travers elle, **caractérisé en ce que** ledit orifice d'entrée (4) passe à travers la surface réfléchissante (5) du miroir (2).

2. Instrument dentaire selon la revendication 1, dans lequel ledit miroir (2) est empêché d'entrer en rotation pendant l'utilisation.

3. Instrument dentaire selon une quelconque des revendications précédentes, dans lequel ledit miroir (2) s'étend comme une bride s'éloignant dudit orifice d'entrée.

4. Instrument dentaire selon une quelconque des revendications précédentes, dans lequel ledit miroir (2) forme une partie intégrante dudit instrument (1).

5. Instrument dentaire selon une quelconque des revendications précédentes, dans lequel ledit miroir (2) est fixable de manière amovible audit instrument (1).

6. Instrument dentaire selon une quelconque des revendications précédentes, dans lequel ledit miroir (2) est un miroir grossissant.

7. Instrument dentaire selon une quelconque des revendications précédentes, dans lequel ledit dispositif d'aspiration (3) comprend une buse d'aspiration tubulaire, dont l'orifice d'entrée forme une bouche (6) et dans lequel ladite buse d'aspiration est incurvée.

8. Instrument dentaire selon la revendication 3, dans lequel ledit miroir (2) est fixé de manière amovible audit dispositif d'aspiration (3) au moyen d'un agencement de couplage par friction (7).

9. Instrument dentaire selon la revendication 3, dans lequel ledit miroir (2) est fixé de manière amovible à un siège dans ledit dispositif d'aspiration (3) au moyen d'un support.
